(19) Europäisches Patentamt
European Patent Office
Office européen des brevets

(11) **EP 3 139 153 A1**

(12) **EUROPÄISCHE PATENTANMELDUNG**

(43) Veröffentlichungstag:
**08.03.2017   Patentblatt 2017/10**

(51) Int Cl.:
*G01N 21/3586* (2014.01)    *G01N 33/34* (2006.01)
*G01N 21/86* (2006.01)    *G01N 21/89* (2006.01)

(21) Anmeldenummer: **15002578.1**

(22) Anmeldetag: **02.09.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(71) Anmelder: **PHILIPPS-UNIVERSITÄT MARBURG**
**D-35032 Marburg (DE)**

(72) Erfinder:
• **Stübling, Eva-Maria**
**34537 Bad Wildungen (DE)**

• **Busch, Stefan F.**
**35037 Marburg (DE)**
• **Gente, Ralf**
**35039 Marburg (DE)**
• **Probst, Thorsten**
**38102 Braunschweig (DE)**

(74) Vertreter: **Stumpf, Peter**
**c/o TransMIT GmbH**
**Kerkrader Strasse 3**
**35394 Gießen (DE)**

Bemerkungen:
Die Patentansprüche wurden nach dem Anmeldetag eingereicht (R. 68(4) EPÜ).

(54) **VERFAHREN UND VORRICHTUNG FÜR DIE THZ-ZEITBEREICHSSPEKTROSKOPIE**

(57)    Die Erfindung beschreibt ein Verfahren und eine Vorrichtung für die THz-Zeitbereichsspektroskopie. Sie erlaubt es insbesondere, den Zeitbereich eines ASOPS-THz-Zeitbereichsspektrometers vollständig auszunutzen, wodurch ortsaufgelöste Messungen mit einer für industrielle Anwendungen erforderlichen Messgeschwindigkeit ermöglicht werden. Ein Puls vom senderseitigen Laser ($L_S$) wird dazu in MxN zeitlich gestaffelte Pulse aufgeteilt, die mit der Probe (Pr) an unterschiedlichen Punkten ortsaufgelöst in Wechselwirkung treten und Signale erzeugen, die entsprechend der zeitlichen Staffelung auf den empfängerseitigen Detektor (E) treffen. Die zeitliche Staffelung der Subpulse erfolgt mit optischen Fasern ($Z_1$-$Z_8$) unterschiedlicher Länge sowie mit weiteren optischen THz-Elementen, z.B. Stufenkeile ($K_1$-$K_8$).

Fig. 4

**Beschreibung**

**Gebiet der Erfindung**

Die Erfindung betrifft ein Verfahren und eine Vorrichtung für die THz-Zeitbereichsspektroskopie, insbesondere die ASOPS-THz-Spektroskopie.

**Stand der Technik**

Der THz-Frequenzbereich ist im elektromagnetischen Spektrum zwischen dem Mikrowellen- und dem infraroten Frequenzbereich lokalisiert und erstreckt sich von ca. 0,1 THz bis 10 THz.

THz-Zeitbereichsspektrometer nutzen zur Messung von THz-Signalen das Prinzip der kohärenten Detektion. Ihr Aufbau und ihre Funktionsweise sind bekannt, umfassend beschrieben z. B. in der EP14156712.3, auf die unten noch näher eingegangen wird. THz-Zeitbereichsspektrometer der 1. Generation nutzen eine mechanische Verzögerungsstrecke mit Freistrahlführung von Laserpulsen im optischen Pfad.

THz-Zeitbereichsspektrometer der 2. Generation verzichten auf eine solche mechanische Verzögerungsstrecke und Freistrahlführung. Die Laserpulse werden in der Regel komplett fasergekoppelt geführt. Um die zum Sampling erforderliche definierte Verzögerung der Pulse zweier Laserpulsfolgen zu realisieren, werden verschiedene andere Verfahren benutzt, welche u.a. als Asynchronous Optical Sampling (ASOPS), Optical Sampling by Cavity Tuning (OSCAT) sowie Electronically Controlled Optical Sampling (ECOPS) bekannt sind. THz-Zeitbereichsspektrometer auf Basis dieser Verfahren sind bereits kommerziell verfügbar.

ASOPS-THz-Spektrometer weisen zwei synchronisierte Femtosekunden-Infarot-Laser (nachfolgend kurz: fs-IR-Laser) auf, deren Repetitionsraten geringfügig, typischerweise um 0,01...0,1 ‰ gegeneinander verstimmt sind. Aus Kostengründen werden Laser mit Repetitionsraten im Bereich 80 MHz ... 250 MHz bevorzugt. Eine typische Kombination wird z. B. gebildet durch einen Senderlaser mit $f_S$ = 250,000 MHz und einen Empfängerlaser mit $f_E$ = 249,975 MHz, wobei es auf eine genaue Einstellung der Verstimmung (Verhältnis $f_E/f_S$) ankommt, nicht auf die Genauigkeit der absoluten Frequenzen.

Die Pulswiederholdauer des Sendelasers, die durch $1/f_S$ gegeben ist, bestimmt die Länge des im Sampling-Verfahren abzutastenden Zeitbereichs Z. Bei $f_S$ = 250 MHz und $f_E/f_S$ = 1,00001 (Verstimmung 0,01 ‰) wird ein 4 ns langer Zeitbereich mit einer Schrittweite von 0,04 ps (100000 Sampling-Punkte) abgetastet, was 400 µs dauert. Verfahrensbedingt ist es bei ASOPS-THz-Spektrometern unvermeidlich, den gesamten Zeitbereich Z abzutasten, obwohl nur in seinem Anfangsbereich ein maximal 100 ps langer THz-Puls enthalten ist, während der übrige, in diesem Fall 3,9 ns lange, Zeitbereich keine Information enthält. In der für das Sampling des THz-Pulses notwendigen Messzeit von 400 µs wird also nur während 10 µs eine relevante Information registriert. Diese unbefriedigende Ausnutzung der Messzeit (hier 2,5 %) ist ein wesentliches Hindernis für den Einsatz von ASOPS-THz-Spektrometern in industriellen Routineverfahren (z. B. Inline-Prüfungen schnell durchlaufender Papierbahnen).

Eine bekannte Lösung für dieses Problem ist das High-Speed-ASOPS, bei dem zwei Laser mit deutlich höherer Repetitionsrate (1 GHz) verwendet werden, beschrieben z. B. in: A. Bartels, T. Dekorsy, Techn. Messen 75 (2008) 1-8. Der Zeitbereich verkürzt sich hier auf $1/f_S$ = Z = 1 ns. Wird er ebenfalls mit einer Schrittweite von 0,04 ps abgetastet, so sind 25000 Sampling-Punkte erforderlich, die in 25 µs registriert werden. Die Messgeschwindigkeit erhöht sich also um den Faktor 16, sie steigt quadratisch mit der Repetitionsrate an. Diese Verbesserung wird aber durch derzeit sehr viel teurere GHz-Laser erkauft und ist daher für industrielle Anwendungen unwirtschaftlich.

Eine Lösung mit vergleichbarer Wirkung, die jedoch auf einer völlig anderen Idee basiert und zu wesentlich niedrigeren Kosten realisierbar ist, beschreibt die nicht vorveröffentlichten europäische Patentanmeldung EP14156712.3, ein unter Art. 54 (3) EPÜ fallendes Dokument, Anmeldetag: 26.02.2014, Offenlegung am 02.09.2015. Die durch Anregung einer THz-Senderantenne mit fs-IR-Laserpulsen von dieser emittierten, divergenten THz-Pulse werden kollimiert und auf eine THz-Optik geleitet, welche sie durch Ausnutzung unterschiedlicher (geometrischer und/oder optischer) Weglängen, die zu definierten Laufzeitdifferenzen führen, in N zeitlich beabstandete THz-Subpulse aufspaltet. Durch diese Folge von N zeitversetzten THz-Subpulsen wird der Messbereich/Zeitbereich eines ASOPS-THz-Spektrometers um einen Faktor N besser ausgenutzt (sofern der Zeitversatz so groß gewählt wird, dass sich die THz-Subpulse nicht überlagern). Werden die N THz-Subpulse, die im kollimierten Bereich auch örtlich separiert sind, durch eine Probe geleitet, so können (in derselben Messzeit wie bei einem herkömmlichen ASOPS-THz-Spektrometer für nur einen Probenort, da nur ein THz-Puls im Zeitbereich enthalten ist) hier Informationen von N Probenorten registriert werden. Es wird somit eine ortsaufgelöste THz-Spektroskopie ermöglicht. Als zwei mögliche Ausführungsformen der Optik zur Herstellung der zeitlich beabstandeten THz-Subpulse werden in der EP14156712.3 ein transmittierender und ein reflektierender Stufenkeil vorgeschlagen.

Die Lösung gemäß der EP14156712.3 beinhaltet bereits einen erheblichen technischen Fortschritt (insb. aufgrund der vernachlässigbar niedrigen Kosten von Stufenkeilen gegenüber GHz-Lasern). Sie weist aber noch folgende Nachteile auf:

Die erreichbare Zeitverzögerung der THz-Subpulse ist begrenzt. Es ist technisch nicht praktikabel, eine Zeitverzögerung von 4 ns, wie sie zur vollständigen Ausnutzung des Zeitbereichs des einleitend skizzierten ASOPS-THz-Spektrometers erforderlich wäre, zu realisieren, denn dazu wäre im Falle eines reflektierenden Stufenkeils eine Stufenkeillänge (Abstand zwischen den beiden äußersten Stufen) von 60 cm, im Falle eines transmittierenden Stufenkeils sogar von 2,4 m erforderlich. (Bei dieser Abschätzung wurde ein Brechungsindex des Stufenkeilmaterials von n = 1,5 angenommen). THz-Spektrometer mit derart langen Stufenkeilen wären unzumutbar sperrig. Limitierend wirkt im Falle eines transmittierenden Stufenkeils zudem dessen hohe Dämpfung im THz-Frequenzbereich, die die Stufenkeillänge auf ca. 30 cm, entsprechend ca. 0,5 ns maximaler Zeitverzögerung, begrenzt. Es ist somit unzweckmäßig, große Zeitverzögerungen von THz-Subpulsen (> 0,5 ns) im THz-Pfad eines THz-Zeitbereichsspektrometers zu realisieren.

Ein weiterer Nachteil besteht darin, dass es an den parallel zur Richtung des THz-Strahls stehenden Stufenkanten zu unerwünschter Streustrahlung kommt, die das Signal/RauschVerhältnis bei spektroskopischen Messungen beeinträchtigt.

Ein dritter Nachteil besteht darin, dass das Strahlprofil des kollimierten THz-Strahls, d. h. seine Intensitätsverteilung quer zu seiner Ausbreitungsrichtung, sehr inhomogen ist. Außenliegende Stufen der Stufenkeile werden mit schwächeren THz-Subpulsen beaufschlagt als innenliegende (d. h. nahe der Mittellinie des des kollimierten THz-Strahls liegende) Stufen. Das Signal/Rausch-Verhältnis bei ortsaufgelösten spektroskopischen Messungen sinkt somit, ausgehend von der Mittellinie, nach außen hin ab.

## Aufgabe der Erfindung

Die Aufgabe der vorliegenden Erfindung besteht darin, die vorangehend genannten Nachteile des Standes der Technik, im Wesentlichen gebildet durch die in der EP14156712.3 beschriebene Lösung, zu überwinden.

Es ist eine Lösung anzugeben, die es erlaubt, auch sehr große Zeitverzögerungen zwischen aus demselben Ausgangssignal erzeugten THz-Subpulsen zu realisieren. Diese Zeitverzögerung soll zumindest 4 ns erreichen (geeignet für ein ASOPS-THz-Spektrometer, ausgestattet mit 250-MHz-Lasern), bevorzugt 10 ns (geeignet für ein ASOPS-THz-Spektrometer, ausgestattet mit 80-MHz-Lasern) erreichen, besonders bevorzugt soll sie praktisch unbegrenzt sein. Ferner soll die erfinderische Lösung spektroskopische Messungen mit einem hohen, über die gesamte Ausdehnung breiter Proben konstanten, Signal/Rausch-Verhältnis gewährleisten. Durch Anwendung der erfinderischen Lösung soll der Zeitbereich eines ASOPS-THz-Spektrometers vollständig ausgenutzt, d.h. mit relevanter Information gefüllt werden.

## Lösung der Aufgabe

Die Aufgabe der Erfindung wird gelöst durch ein Verfahren, bei dem bereits im optischen Pfad eines THz-Spektrometers eine Zerlegung der von einem von einem fs-IR-Laser erzeugten IR-Anregungspulse in M identische zeitversetzte IR-Subpulse erfolgt (mit großem Zeitversatz, Grobzerlegung), welche dann auf M THz-Senderantennen geführt werden und diese jeweils zur Emission eines THz-Pulses anregen. Insgesamt werden somit M THz-Pulse mit großem Zeitversatz erhalten, die sich auf räumlich separierten Strahlwegen ausbreiten. Jeder THz-Puls wird dann durch eine THz-Optik, wie aus der EP14156712.3 bekannt, in N zeitversetzte THz-Subpulse (mit kleiner Zeitverzögerung, Feinzerlegung) zerlegt. Aus jedem IR-Anregungspuls resultieren somit zeitversetzte MxN THz-Subpulse. Zur Anwendung in eine ASOPS-THz-Spektrometer, gekennzeichnet durch einen Zeitbereich Z, wird die Zeitverzögerung aufeinanderfolgender THz-Pulse auf einen Wert $Z/M \times N$ eingestellt, wodurch der Zeitbereich vollständig mit $M \times N$ zeitlich äquidistanten THz-Subpulsen gefüllt wird.

Ferner wird die Aufgabe der Erfindung gelöst durch die Angabe von technischen Vorrichtungen zur Realisierung des erfindungsgemäßen Verfahrens aus Grob- und Feinzerlegung. Eine erste Vorrichtung, geeignet für die Grobzerlegung im optischen Pfad, beinhaltet einen fs-IR-Laser, der mit M Zero-Dispersion-Fibers zum Transport von IR-Lasersignalen ausgestattet ist, die (zum Zwecke der Realisierung großer Zeitverzögerungen) definierte, unterschiedliche Längen aufweisen und jeweils mit einer (von insgesamt M) THz-Senderantenne verbunden sind. Eine zweite Vorrichtung, geeignet für die Feinzerlegung im THz-Pfad, betrifft eine THz-Optik, umfassend mehrere Blöcke aus für THz-Strahlung transparentem Material, an deren Oberflächen ein einfallender THz-Strahl teilweise reflektiert wird, wodurch aus einem THz-Puls N definiert zeitversetzte, auf parallelen, definierte räumliche Abstände aufweisenden, Strahlwegen verlaufenden, THz-Subpulse generierbar sind. Diese Vorrichtung kann auch mit einem THz-Strahl mit geringem Strahldurchmesser betrieben werden, sie benötigt keinen aus einem divergenten Antennensignal generierten, kollimierten THz-Strahl mit großem, der Breite einer zu untersuchenden Probe entsprechendem, Strahlquerschnitt, wie er für die aus dem Stand der Technik (EP14156712.3) bekannten Stufenkeile erforderlich ist.

## Detaillierte Darstellung der erfindungsgemäßen Lösung

Um die Zeitverzögerung von aus einem THz-Puls erzeugten, zeitversetzten THz-Subpulsen auf ca. 0,5 ns (zwischen dem ersten und dem letzten THz-Subpuls) zu begrenzen, dennoch aber THz-Subpulse mit größeren Zeitversätzen zu erzeugen, wird erfindungsgemäß wie folgt vorgegangen: Es wird bereits jeder von einem fs-IR-Anregungslaser erzeugte IR-Puls einer Aufspaltung unterzogen und in M IR-Subpulse gleicher Intensität und Form zerlegt, die jeweils in eine von M Zero-Dispersion-Fibers (nachfolgend ZDFs, Singular: ZDF), welche mit jeweils einer von M THz-Senderantennen verbunden ist, eingeleitet werden. Eine ZDF zeichnet sich dadurch aus, dass die in ihr transportierten Laserpulse (auf den hier relevanten Strecken in der Größenordnung von 1 m) weder nachweisbar verformt noch gedämpft werden. Die IR-Subpulse behalten beim Durchlaufen der ZDFs somit ihre Intensität und ihre Pulsdauer, die im Bereich von 100 fs liegt, bei.

Die gewünschten großen Zeitverzögerungen der IR-Subpulse, z. B. Zeitverzögerungen von 0,5 ns und ganzzahligen Vielfachen dieses Werts, sind durch den Einsatz von ZDFs geeigneter Länge, die entsprechende Laufzeitdifferenzen, z. B. $(i - 1)\,0{,}5$ ns $(i = 1... M)$, gewährleisten, problemlos herstellbar.

Die Längen der ZDFs sind wie folgt zu berechnen: Der zu füllende Zeitbereich Z wird durch die Anzahl M der eingesetzten THz-Antennen dividiert, wodurch sich die Zeitverzögerung $\Delta T = Z/M$ zweier aufeinanderfolgender, zur seriellen Anregung zweier THz-Antennen genutzter, IR-Subpulse ergibt. Diese Zeitverzögerung wird realisiert durch eine entsprechende Längendifferenz $\Delta L$ der zugehörigen ZDFs:

$$\Delta L = (c/n) \cdot \Delta T$$

Dabei sind c die Vakuumlichtgeschwindigkeit und n der Brechungsindex des Fasermaterials, aus dem die ZDF hergestellt ist. Zu berücksichtigen ist noch die Länge $L_0$ der kürzesten ZDF, die zur nächstgelegenen THz-Antenne führt.

Die Faserlängen $L_i$ der ZDFs, die den fs-IR-Laser mit den M THz-Senderantennen verbinden, sind somit gegeben durch:

$$L_i = L_0 + (i - 1) \cdot \Delta L \text{ mit } \Delta L = (c/n) \cdot Z/M ,$$

wobei der Index i die Werte 1 bis M durchläuft.

Es ist möglich, eine begrenzte, für alle ZDFs identische, Dispersion in Kauf zu nehmen und den für alle ZDFs gleich langen Abschnitt $L_0$ nicht aus ZDF-Material auszuführen, sondern dafür eine (kostengünstigere) herkömmliche Faser zu verwenden. Nur die über $L_0$ hinausgehenden Abschnitte müssen aus ZDF-Material ausgeführt werden.

Zu beachten ist, dass $L_0$ nicht beliebig klein gewählt werden kann. Werden nämlich sehr breite Proben (Probenbreite in der Größenordnung von 1 m und mehr) mit einer entsprechend breiten Anordnung aus mehreren THz-Senderantennen untersucht, so können die Antennenabstände größer sein als die Längendifferenz $\Delta L$ der ZDFs. Wird die dem Laser nächstgelegene Antenne mit einer ZDF angeschlossen, die eine zu geringe Länge $L_0$ aufweist, so sind die weiter entfernt positionierten Antennen durch die ihnen zugeordneten ZDFs nicht erreichbar. Es ist daher zweckmäßig, den fs-IR-Laser nahe der Mitte der Antennenanordnung zu positionieren und zunächst die beiden äußeren Antennen mit hinreichend langen, eine Längendifferenz $\Delta L$ aufweisenden, ZDFs anzuschließen. Nach innen hin sind die weiteren Antennen dann problemlos durch jeweils um $\Delta L$ kürzer gewählte ZDFs problemlos anzuschließen.

Werden die so aus einem IR-Laserpuls erzeugten zeitversetzten IR-Subpulse zur Anregung von M THz-Senderantennen genutzt, so füllen die emittierten M THz-Pulse, wenn sie in ein ASOPS-Spektrometer eingespeist werden, dessen Zeitbereich gleichmäßig in äquidistanten Abständen. Durch diese Grobzerlegung wird jedoch, im Vergleich zur EP14156712.3, noch keine grundsätzliche Verbesserung der Ausnutzung dieses Zeitbereichs erzielt, denn die M THz-Pulse sind jetzt zwar gleichmäßig auf den Zeitbereich/Messbereich verteilt, jedoch verbleiben zwischen ihnen weiterhin zeitlich ausgedehnte informationslose Zwischenräume. Diese Lösung wäre lediglich teurer, da anstelle eines kostengünstigen Stufenkeils weitere THz-Antennen benötigt werden.

Der entscheidende erfindungsgemäße Fortschritt wird erzielt, indem die vorab beschriebene Grobzerlegung jedes IR-Pulses durch eine Feinzerlegung der zugehörigen THz-Pulse ergänzt wird. Jeder der M THz-Pulse wird dabei in jeweils N zeitversetzte THz-Subpulse aufgespalten. Der Zeitversatz $\Delta t$ aufeinanderfolgender THz-Subpulse ergibt sich dabei durch:

$$\Delta t = Z/(M \times N)$$

Somit wird, bei Anwendung in einem ASOPS-Spektrometer, dessen Zeitbereich Z mit $M \times N$ THz-Subpulsen $P_{i,j}$ gefüllt, deren Zeitversätze $T_{i,j}$ gegeben sind durch

$$T_{i,j} = (i - 1) \cdot Z/M + (j - 1) \cdot Z/(M \times N) ,$$

wobei der Index i die Werte 1 bis M, der Index j die Werte von 1 bis N durchläuft.

Die THz-Subpulse sind äquidistant über den gesamten Zeitbereich Z geteilt. Ihre Anzahl kann (durch Auswahl einer passenden Anzahl N unter Berücksichtigung der auswertbaren Länge der THz-Subpulse, d. h. der Zeit bis zum Abklingen eines solchen Pulse auf null) so gewählt werden, dass sie den Zeitbereich lückenlos (ohne informationslose Zwischenräume zwischen den THz-Pulsen) oder nahezu lückenlos füllen.

Hat die auswertbare Länge der THz-Subpulse den Wert $\tau$, so lässt sich im Zeitbereich Z eine Anzahl $P_{ges} = Z/\tau$ von THz-Subpulsen unterbringen. Auf jede der M THz-Senderantennen entfallen somit $P_A = Z/(M \cdot \tau)$ THz-Subpulse. Ist $P_A$ ganzzahlig, so wird für N dieser Wert gewählt.

Ist $P_A$ nicht ganzzahlig, so wird die nächstniedrigere ganze Zahl als Wert für N gewählt. Dadurch wird vermieden, dass sich aufeinanderfolgende THz-Subpulse in unerwünschter Weise teilweise überlagern. Stattdessen verbleibt ein kurzer informationsloser Zwischenraum zwischen diesen THz-Subpulsen.

Bei den meisten Anwendungen beträgt die maximal auswertbare Länge der THz-Subpulse 100 ps. Oft liegt sie deutlich niedriger, z. B. bei 20 ps bis 60 ps, bei speziellen Anwendungen wie z. B. Compressed Sensing, noch darunter.

Zur Realisierung der Zeitversätze, die für aufeinanderfolgende THz-Subpulse der auswertbaren Pulslänge $\tau$ entsprechen oder geringfügig größer sind, stehen die aus der EP14156712.3 bekannten Stufenkeile zur Verfügung, deren Stufenhöhen H zu wählen sind gemäß der Formel H = c·$\tau$/2 für einen reflektierenden bzw. H = c·$\tau$/(n - $n_U$) für einen transmittierenden Stufenkeil, wobei n der Brechungsindex des Stufenkeilmaterials und $n_U$ der Brechungsindex des umgebenden Mediums, in der Regel Luft, also $n_U$ =1, ist. Für $\tau$ ist, wenn $P_A$ ganzzahlig ist, unmittelbar die auswertbare Länge der THz-Subpulse einzusetzen. Ist $P_A$ nicht ganzzahlig, so ist $\tau$ durch einen geringfügig größeren Wert zu ersetzen, der sich ergibt, wenn in der Formel $P_A$ = Z/(M·$\tau$) der nicht ganzzahlige Wert von $P_A$ durch die nächstniedrigere ganze Zahl ersetzt wird.

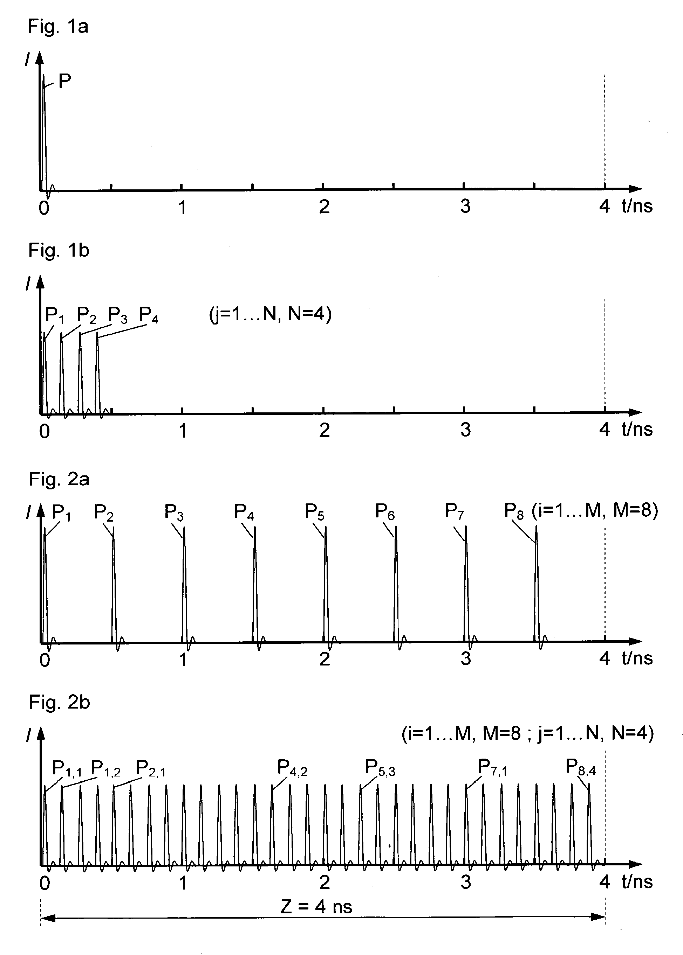

Beispielhaft zusammengefasst wird das beschriebenen Verfahren und der damit erzielte Fortschritt in den Figs. 1 und 2, die die Füllung eines 4 ns langen Zeitbereichs Z mit THz-Pulsen bei verschiedenartig ausgeführten ASOPS-THz-Spektrometern veranschaulichen. Die Indizierung der Pulse erfolgt wie oben erläutert.

Fig. 1a zeigt den Zeitbereich eines herkömmlichen ASOPS-THz-Spektrometers, der nur am Anfang mit einem einzigen THz-Puls P gefüllt ist. Fig. 1b zeigt den durch die EP14156712.3 erzielten Fortschritt. Durch Aufspaltung des von einer THz-Antenne emittierten THz-Pulse in N = 4 zeitversetzte THz-Subpulse $P_1$, $P_2$, $P_3$, $P_4$, z. B. mithilfe eines 4-stufigen Stufenkeils, wird der Zeitbereich Z um den Faktor N=4 besser ausgenutzt.

Fig. 2a zeigt das Resultat der Grobzerlegung eines fs-IR-Laserpulses: M=8 THz-Antennen werden zeitversetzt zur Emission von 8 THz-Pulsen $P_1$, $P_2$,..., $P_8$ angeregt, die den Zeitbereich in äquidistanten Abständen füllen, wobei zwischen den THz-Pulsen noch lange informationslose Zwischenräume verbleiben. Fig. 2b zeigt das Ergebnis der erfindungsgemäßen Kombination aus Grob- und Feinzerlegung: Jeder THz-Puls wird in N=4 zeitversetzte THz-Subpulse aufgespalten, sodass der Zeitbereich nun vollständig gefüllt ist und zur Erfassung von Probeninformationen hier MxN=32 Messflecke, denen (bei Vorhandensein einer Probe) Probenorte zugeordnet sind, genutzt werden können. Zwecks besserer Visualisierung wurde in den Figs. 1 und 2 ein kleiner Wert für N (N=4) gewählt. N kann deutlich größer sein, z. B., wie im später folgenden Ausführungsbeispiel, N = 10. Aus Platzgründen wurden in Fig. 2b nur ausgewählte Pulse indiziert. Die fehlenden Indizes sind problemlos zu ermitteln. Prinzipiell kann die Anzahl N, z. B. durch Verwendung eines Stufenkeils mit der entsprechenden größeren Anzahl von Stufen N, beliebig vergrößert werden, sodass Informationen an mehr, aber kleineren Messflecken, denen Probenorte zugeordnet sind, gewonnen wird, wodurch sich die Ortsauflösung verbessert. Jedoch geschieht das zu Lasten der spektralen Auflösung und des Signal/Rausch-Verhältnisses der Messungen. Der Fachmann ist in der Lage, hier einen geeigneten Kompromiss zu finden. Er kann beispielsweise auch mehrere Einzelmessungen akkumulieren. Die zur Untersuchung einer Probe verfügbaren Messflecke können auf einer geraden Linie angeordnet sein, sie können aber auch, z. B. durch Verwendung eines zweidimensionalen Stufenkeils, d. h. eines Stufenkeils, bei dem die Stufen matrixartig auf einer Fläche angeordnet sind, auf einer Fläche verteilt sein. Die Verwendung solcher Stufenkeile ist in der EP14156712.3, Ausführungsbeispiel 5, S. 24, Z. 1-12, beschrieben. Ebenso besteht die Möglichkeit, die Anzahl M der THz-Senderantennen zu erhöhen und dadurch die Breite des von einer THz-Senderantenne zu überwachenden Probenabschnitts zu verringern. Dazu ist sicherzustellen, dass die Leistung des fs-IR-Anregungslasers ausreicht, um auch die größere Anzahl von THz-Senderantennen anzuregen. Die Zuordnung von Messflecken zu Probenorten ist ebenfalls in der EP14156712.3 beschrieben (S. 9, Z. 7-12, S. 9, Z. 31 - S. 10, Z. 2), wobei Probenorte dort als Probenabschnitte bezeichnet sind.

Als Alternative zu den bekannten Stufenkeilen wird nachfolgend eine neuartige THz-Optik vorgeschlagen, die die im Stand der Technik genannten Nachteile der Stufenkeile überwindet.

Die neuartige Optik weist eine Anordnung von HDPE-Blöcken (HDPE = High density polyethylene, Polyethylen hoher Dichte) auf, durch die hindurch der von einer THz-Senderantenne emittierte THz-Puls sich ausbreitet. HDPE zeichnet sich durch eine geringe Absorption im THz-Frequenzbereich aus und ist daher ein bevorzugtes Material für THz-Linsen. Die HDPE-Blöcke sind so ausgeführt, dass sie an ihren Oberflächen (bei Eintritt in einen und Austritt aus einem Block) jeweils einen definierten Anteil des einlaufenden THz-Pulses reflektieren und dadurch je Block zwei definiert zeitversetzte THz-Subpulse generieren. Der an einer Oberfläche reflektierte Anteil r liegt zwischen 1 % und 10 %, vorzugsweise bei 5 %. Der wesentlich größere, in seiner Intensität nahezu unveränderte, nichtreflektierte Anteil des einlaufenden THz-Pulses schreitet durch die Blockanordnung hindurch fort. Die Anzahl B der Blöcke, ihre Geometrie (die Dicke, die Neigung der Oberflächen gegenüber dem THz-Strahl, welche Einfalls-, Ausfalls- und Reflexionswinkel bestimmt), sowie die Blockanordnung, insbesondere der Blockabstand, werden so gewählt, dass die gewünschte Zahl N = 2B parallel verlaufender THz-Subpulse erhalten wird, welche einen an die Breite des zu untersuchenden Probenabschnitts angepassten äquidistanten räumlichen Abstand sowie einen an die auswertbare Pulslänge angepassten Zeitversatz aufweisen. Da an einer Oberfläche nur ein geringer Anteil r des einlaufenden THz-Pulses reflektiert wird, sinkt dessen Intensität nur langsam ab und alle N THz-Subpulse weisen ungefähr gleiche Intensitäten auf.

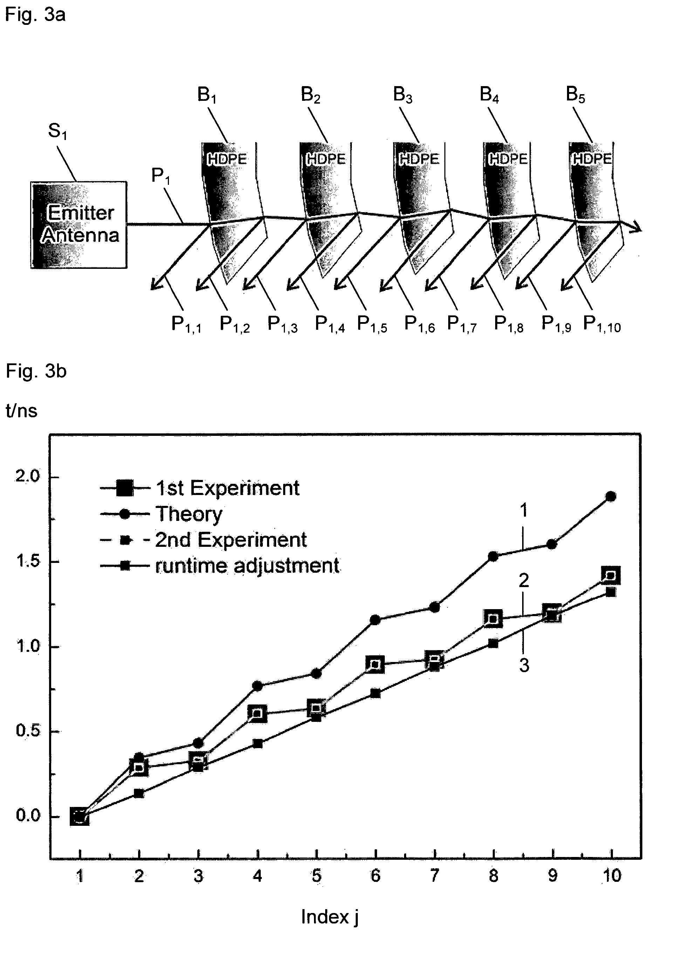

Eine schematische Darstellung der neuartigen Optik und ihrer Funktionsweise zeigt Fig. 3a. Die Optik weist 5 HDPE-Blöcke $B_{1,...}B_5$ auf. Sie wird durch eine THz-Senderantenne $S_1$ mit einem THz-Puls $P_1$ beaufschlagt, der die Blockanordnung durchläuft. Durch Reflexion an den Oberflächen der Blöcke werden 10 räumlich äquidistant beabstandete parallel verlaufende THz-Subpulse $P_{1,1}$, $P_{1,2}$, ..., $P_{1,10}$ generiert.

Da die Zeitverzögerungen aufeinanderfolgender THz-Subpulse leicht variieren, wird die Anordnung der HDPE-Blöcke mit einem (nicht dargestellten) Spiegelsystem aus N Spiegeln ausgestattet. Jeder THz-Subpuls wird durch einen in in definiertem Abstand positionierten Spiegel reflektiert, wodurch die THz-Subpulse äquidistante zeitliche Abstände erhalten. Fig. 3b zeigt experimentell erhaltene, sehr gute Ergebnisse der Linearisierung der zeitlichen Abstände der THz-Subpulse. Dabei sind die Zeitverzögerungen über dem von 1 bis 10 laufenden Index j der THz-Subpulse aufgetragen. Kurve 1 zeigt berechnete Zeitverzögerungen, Kurve 2 zeigt die in zwei Experimenten, deren Ergebnisse visuell ununterscheidbar sind, gemessenen Zeitverzögerungen, beides ohne Korrektur durch das Spiegelsystem. Kurve 3 zeigt die mithilfe des Spiegelsystems ausgezeichnet linearisierten Zeitverzögerungen, ebenfalls experimentell bestimmt.

Die neuartige THz-Optik kann, wie Fig. 3a verdeutlicht, mit einem THz-Strahl sehr kleinen Durchmessers betrieben werden und führt die erforderliche Separation in räumlich getrennte THz-Subpulse dann selbst herbei. Sie bietet somit einen wesentlichen Vorteil gegenüber den aus der EP14156712.3 bekannten Stufenkeilen, die bereits mit einem räumlich aufgeweiteten THz-Strahl beaufschlagt werden müssen. Ein weiterer Vorteil besteht in der im Vergleich zu einem transmittierenden Stufenkeil niedrigeren Absorption. Ein dritter Vorteil besteht darin, dass keine Streustrahlung infolge von parallel zum THz-Strahl verlaufenden Kanten auftritt.

**Ausführungsbeispiel 1**

Das erfindungsgemäße Verfahren und die erfindungsgemäße Vorrichtung werden genutzt, um den Zeitbereich eines ASOPS-Spektrometers vollständig auszunutzen und es zur Überwachung von 4 m breiten Papierbahnen einzusetzen.

Das ASOPS-Spektrometer ist mit zwei fs-IR-Lasern ausgestattet, wobei der senderseitige Laser eine Repetitionsrate $f_S$ = 250,0000 MHz aufweist.

Der Zeitbereich Z = 4 ns soll mit $10^5$ Messpunkten im äquidistanten Abstand von 40 fs abgetastet werden. Dazu wird der empfängerseitige Laser um 0,01 ‰ verstimmt und somit auf eine Repetitionsrate $f_E$ = 249,9975 MHz eingestellt. Wie in der Beschreibung erläutert, kommt es dabei nur auf die präzise Einstellungen des Quotienten $f_E/f_S$ an, der hier 1,00001 beträgt, die Absolutwerte $f_S$ und $f_E$ können proportional zueinander leicht verschoben sein. Die zur Abtastung des Zeitbereichs erforderliche Messzeit beträgt somit $10^5$ 4 ns = 400 µs.

Die auswertbare Länge τ der THz-Subpulse beträgt 50 ps, d. h. nach 50 ps ist der Puls auf null abgeklungen, der nächste Puls kann direkt angeschlossen werden. Somit sind in den Zeitbereich 80 THz-Subpulse einzuspeisen, die äquidistant durch jeweils 1250 Sampling-Punkte abzutasten sind.

Da eine THz-Senderantenne lediglich eine Ausgangsleistung liefert, die zur Überwachung einer maximal 50 cm breiten Probe ausreicht, werden 8 THz-Senderantennen verwendet. Durch den senderseitigen fs-IR-Laser werden diese 8 THz-Antennen seriell mit einem Zeitversatz von 500 ps (zwischen zwei aufeinanderfolgend angeregten THz-Senderantennen) zur Emission von THz-Pulsen angeregt, indem 8 aus einem IR-Puls erhaltene identische IR-Subpulse über ZDFs unterschiedlicher Länge zu den Antennen geleitet werden. Bei einem Brechungsindex des Fasermaterials der ZDF von n = 1,5 ergibt sich eine Längendifferenz der ZDFs von 10 cm. Bei einem entsprechend der Geometrie zu wählenden Wert $L_0$ für die kürzeste Faser betragen die Längen $L_i$ der ZDFs also $L_0$, $L_0$ + 10 cm, ..., $L_0$ + 70 cm.

Jede THz-Antenne ist einem 50 cm breiten Probenabschnitt zugeordnet, in dessen Mittelebene sie positioniert wird. (Die Mittelebene ist eine Ebene, die senkrecht auf dem Probenabschnitt, hier einem Teil einer Papierbahn, steht und dessen Mittellinie schneidet.) Die Antennenanordnung hat somit eine Breite von 3,5 m, wobei die äußeren Antennen jeweils 25 cm vom Probenrand entfernt sind (d. h. der auf die Antennenanordnung projizierte Abstand beträgt 25 cm).

Zweckmäßigerweise wird der fs-IR-Laser in 5 cm Abstand zur Mittelebene der 4 m breiten zu überwachenden Papierbahn positioniert, wobei sein Abstand zu der Linie, auf der die THz-Senderantennen angeordnet sind, möglichst gering, bevorzugt wenige cm, gewählt wird. Seine auf die Antennenanordnung projizierten Abstände zu den beiden äußeren THz-Senderantennen betragen dann 1,80 m sowie 1,70 m. Die beiden äußeren Antennen werden dann durch zwei hinreichend lange ZDFs, deren Längen sich um 10 cm unterscheiden, angeschlossen. Die 1,80 m entfernte THz-Senderantenne wird mit einer 2,00 m langen ZDF, die 1,70 m entfernte THz-Senderantenne wird mit einer 1,90 m langen ZDF angeschlossen. Nach innen hin werden die weiteren THz-Senderantennen nun problemlos durch jeweils um 10 cm kürzer werdende ZDFs angeschlossen (Längen dieser ZDFs: 1,80 m, 1,70 m,..., 1,30 m). Die Länge $L_0$ der kürzesten Faser beträgt hier somit 1,30 m. Wenn die Geometrie es erfordert, kann $L_0$ auch größer gewählt

werden, wenn die Geometrie es zulässt, kann $L_0$ auch noch etwas kleiner gewählt werden. $L_0$ muss aber stets größer als 1,10 m sein, da sonst die außenliegenden THz-Senderantennen durch ihre ZDFs, deren Längen natürlich der $L_0$-Änderung entsprechend anzupassen sind, nicht erreicht werden könnten. Das beschriebene Vorgehen stellt sicher, dass ein für die Ankopplung aller THz-Senderantennen ausreichender Wert für $L_0$ gewählt wird.

Jeder THz-Senderantenne ist eine THz-Optik zugeordnet, die eine Feinaufspaltung des von der THz-Senderantenne emittierten THz-Pulses in 10 THz-Subpulse, die jeweils um 50 ps zeitversetzt sind, realisiert. Als Optik wird die neuartige, auf HDPE-Blöcken basierende Anordnung gewählt. Die Optik weist 5 PE-Blöcke auf, sodass durch Reflexion an ihren Vorder- und Rückseiten 10 zeitlich und räumlich versetzte THz-Subpulse erzeugt werden.

Durch Wahl eines geeigneten Einfallswinkels wird der räumliche Versatz so eingestellt, dass die THz-Subpulse dieser THz-Antenne eine Probenbreite von 50 cm abdecken. Durch den modularen Aufbau mit 8 THz-Senderantennen in Verbindung mit einer solchen THz-Optik wird die spektroskopische Untersuchung von 4 m breiten Proben realisiert. In derselben Messzeit, in der ein herkömmliches ASOPS-Spektrometer einen Probenpunkt erfasst, werden hier 80 Probenpunkte, die sich an verschiedenen Orten befinden, erfasst.

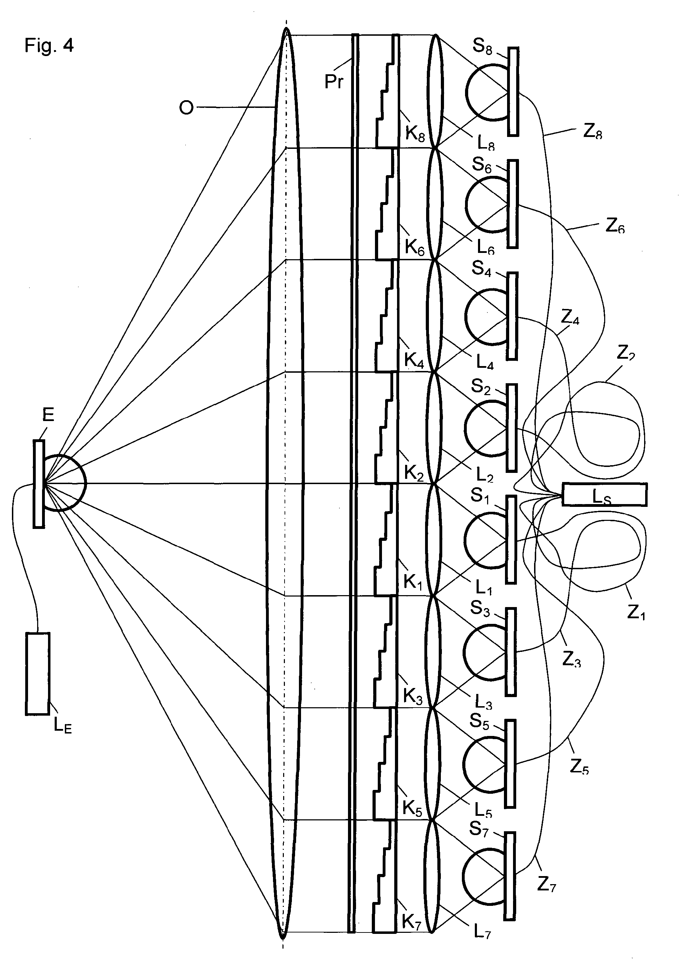

Eine schematische Darstellung der im Ausführungsbeispiel verwendeten Anordnung eines ASOPS-THz-Spektrometers zeigt Fig. 4. Sie umfasst senderseitig einen fs-IR-Laser $L_S$, der über 8 ZDFs $Z_1,...,Z_8$ mit 8 linear nebeneinander angeordneten THz-Senderantennen $S_1,...S_8$ verbunden ist. Der fs-IR-Laser $L_S$ ist um 5 cm gegen die Mittelebene der Anordnung, die insgesamt 4 m breit ist, verschoben. Die Antennen sind in der Reihenfolge der Länge ihrer ZDFs indiziert. Aus Gründen der Übersichtlichkeit sind, anstelle der im Ausführungsbeispiel verwendeten Optik mit HDPE-Blöcken, Linsen $L_1,...,L_8$ und Stufenkeile $K_1,...,K_8$ dargestellt, die ebenfalls verwendbar sind. Linsen und Stufenkeile bezeichnen in dieser Anmeldung stets Komponenten, die für den THz-Frequenzbereich geeignet sind, also eine niedrige Absorption in diesem Bereich aufweisen. Sie werden daher aus einem Material mit niedrigem Absorptionskoeffizienten im THz-Frequenzbereich hergestellt, z. B. ebenfalls aus HDPE.

Werden Stufenkeile verwendet, so können sowohl Stufenkeile mit nur einer Reihe von linear angeordneten Stufen gewählt werden, um entlang einer Linie positionierte Messflecke bereitzustellen. Es können aber auch zweidimensionale Stufenkeile (oben beschrieben) verwendet werden, um zweidimensional auf einer Fläche verteilte Messflecke bereitzustellen.

Letzteres eröffnet die Möglichkeit, einen auf einer bewegten Probe detektierten Fehler nachzufolgen, also ihn entlang seiner Bahn an mehreren Messflecken zu detektieren und dadurch sicherer nachzuweisen.

Die von der Anordnung erzeugten zeit- und ortsversetzten $TH_z$-Subpulse, die nicht dargestellt sind, durchlaufen eine Probe Pr, die nicht Teil des Spektrometers ist, und werden dann durch eine empfängerseitige Optik O auf eine THz-Empfängerantenne E fokussiert, die sie in äquidistanten Zeitabständen erreichen. Die empfängerseitige Optik O ist hier symbolisch als Linse dargestellt, deren Durchmesser die gesamte Ausdehnung der Anordnung abdeckt. In der Praxis sind jedoch mehrkomponentige Optiken zu bevorzugen. Beispielsweise kann zur Fokussierung der von jedem Probenabschnitt (d. h. den hier 50 cm breiten Abschnitten, die jeweils mit den THz-Subpulsen einer bestimmten THz-Senderantenne beaufschlagt werden) gelieferten Information (in Form von beim Durchlaufen der Probe modifizierten THz-Subpulsen) jeweils eine separate Linse mit einem der Breite des Probenabschnitts angepassten Durchmesser oder ein Linsensegment (in Form eines Linsenausschnitts dieser Abmessung) verwendet werden. Die Fokussierung kann direkt auf die THz-Empfängerantenne E erfolgen. Sie kann aber auch stufenweise über weitere zwischengeschaltete optische Elemente erfolgen, bevor abschließend die Fokussierung auf die THz-Empfängerantenne E erfolgt. Anstelle von Linsen und Linsenausschnitten können auch Spiegel und Spiegelausschnitte, insbesondere die für Anwendungen im THz-Frequenzbereich häufig verwendeten Parabolspiegel und Parabolspiegelausschnitte, eingesetzt werden.

Die THz-Empfängerantenne E wird durch fs-IR-Pulse eines zweiten, wie beschrieben verstimmten, fs-IR-Lasers $L_E$ in durch seine Pulswiederholdauer gegebenen Zeitabständen kurzzeitig (für wenige fs) zur Registrierung eines Sampling-Punkts freigeschaltet. Zum Zwecke der Synchronisation beider fs-IR-Laser sind diese mit einer (nicht dargestellten) Steuer-, Mess- und Auswerteelektronik verbunden. Ebenso sind alle Antennen mit dieser Steuer-, Mess- und Auswerteelektronik verbunden.

## Ausführungsbeispiel 2

Im vorangehenden Ausführungsbeispiel 1 werden die innen liegenden THz-Senderantennen (in Fig. 4 mit $S_1$ und $S_2$ bezeichnet) aufgrund der Ankopplung mit den kürzesten ZDFs zuerst zeitversetzt angeregt, dann setzt sich die Anregung zu den weiter außen liegenden Antennen hin fort. Es ist zu beachten, dass die Reihenfolge der Anregung der THz-Senderantennen und die Zeitverzögerung der Anregung natürlich beliebig gewählt werden können, indem die Längen der ZDFs angepasst werden. Bei der Wahl der Reihenfolge und der Zeitverzögerungen sind auch die Anordnung und Ausführung der Komponenten auf der Empfängerseite des Spektrometers, insb. die Position der Empfängerantenne E und die Eigenschaften der Optik O (Brechungsindex, Dickenprofil), die THz-Subpulse nach Durchlaufen des Probenbereichs auf die THz-Empfängerantenne fokussiert, zu berücksichtigen. Reihenfolge der Anregung und Zeitverzögerungen müssen so gewählt werden, dass an der Empfängerantenne E eine möglichst lückenlose Folge von sich nicht überlagernden THz-Subpulsen eintrifft, wie sie in Fig. 2b dargestellt ist. Dazu kann es, z. B. wenn die THz-Empfängerantenne E wie in Fig. 4 in der Mittelebene der Anordnung positioniert ist, notwendig sein, die Reihenfolge der Antennenanregung zu ändern, indem z. B. die äußeren Antennen zuerst angeregt werden und sich die Anregung dann in Richtung Mitte hin fortsetzt. Dazu kann bei den beiden äußeren Antennen die Länge ihrer ZDFs (1,90 m und 2,00 m) beibehalten werden, nach innen sind die ZDF-Längen dann in Schritten von 10 cm zu vergrößern: 2,10 m,..., 2,60 m. $L_0$ beträgt in diesem Fall also 1,90 m. (Diese abgeänderte Anordnung wird ebenfalls durch Fig. 4 repräsentiert, lediglich die Indizes der ZDFs, THz-Senderantennen, Linsen und Stufenkeile wären wie folgt abzuändern:

$7 \rightarrow 1$,
$8 \rightarrow 2$,
$5 \rightarrow 3$,
$6 \rightarrow 4$,
$3 \rightarrow 5$,
$4 \rightarrow 6$,
$1 \rightarrow 7$,
$2 \rightarrow 8$.

Zur Feinanpassung an die genaue Position der Empfängerantenne E und die Eigenschaften der Optik O können weitere individuelle Längenanpassungen der ZDFs vorgenommen werden. Eine weitere Möglichkeit zur Feinanpassung besteht in einer (für jede THz-Senderantenne separat wählbaren) Verschiebung der THz-Senderantennen in Ausbreitungsrichtung der THz-Subpulse. Eine dritte Möglichkeit zur Feinanpassung bietet die Anpassung der Strahlführung auf der Empfängerseite, also die konkrete Ausgestaltung der Optik O. Somit stehen dem Fachmann zumindest drei "Stellschrauben" zur Verfügung, um die Empfängerantenne E mit einer möglichst lückenlosen Folge von sich nicht überlagernden (gleichsam wie an einer Perlenkette aufgereihten) THz-Subpulsen zu beaufschlagen.

In der Beschreibung und den Ausführungsbeispielen ist die senderseitige Ausführung eines ASOPS-THz-Spektrometers mit der erfindungsgemäßen Vorrichtung vollständig beschrieben: Sie umfasst einen fs-IR-Laser, eine geeignete Anzahl M von durch ihn über faserkoppelt (mittels ZDF) übertragbare zeitversetzte fs-IR-Subpulse (mit großem Zeitversatz, Grobzerlegung) zur Emission von THz-Pulsen anregbaren THz-Senderantennen sowie M jeweils einer THz-Senderantenne zugeordnete THz-Optiken zur Feinzerlegung jeweils eines THz-Pulses in zeitversetzte THz-Subpulse. Ebenso ist das von der Vorrichtung realisierbare Verfahren umfassend beschrieben. Es ermöglicht die Nutzung des gesamten Zeitbereichs eines ASOPS-THz-Spektrometers. Zudem wurden auch mehrere Möglichkeiten zur empfängerseitigen Ausführung des ASOPS-THz-Spektrometers präsentiert, insbesondere zur Ausführung der Optik O.

Die Steuer-, Mess- und Auswerteelektronik stellt die Synchronisation beider fs-IR-Laser mit geringfügiger konstanter Verstimmung sicher und wertet die gewonnenen Probeninformationen in Echtzeit aus, sodass eine unmittelbare Reaktion auf registrierte Fehler in der Probe gewährleistet wird.

Es ist an dieser Stelle nicht notwendig, die Steuer-, Mess- und Auswerteelektronik, insbesondere deren Verbindung mit den beiden fs-IR-Lasern, den THz-Senderantennen und der THz-Empfängerantenne näher zu beschreiben, da entsprechende Informationen bereits der EP14156712.3, Ausführungsbeispiel 4, S. 21, Z. 31 - S. 22, Z. 3, sowie Fig. 6a, zu entnehmen sind.

Die gegebenen Informationen erlauben es dem Fachmann somit, die Erfindung auszuführen und ein erfindungsgemäßes ASOPS-THz-Spektrometer, gekennzeichnet durch eine vollständige Ausnutzung seines Zeitbereichs, zu bauen und zu betreiben.

Es ist dabei nicht notwendig, die zur Anregung der THz-Senderantennen und der THz-Empfängerantenne verwendeten fs-IR-Laser auf solche mit moderaten Pulswiederholraten (80 MHz bis 250 MHz) einzuschränken. Insbesondere kann das erfindungsgemäße ASOPS-THz-Spektrometer auch mit Lasern höherer Pulswiederholrate, z. B. 1 GHz oder höher, kombiniert werden. Dadurch wird die Erfindung mit dem im Stand der Technik zitierten High-Speed-ASOPS kombiniert, wodurch, allerdings mit hohen Kosten verbunden, die Messgeschwindigkeit noch gesteigert wird.

Die Erfindung schafft somit die Voraussetzungen für den Routineeinsatz von ASOPS-THz-Spektrometern in industriellen Fertigungsprozessen, z. B. zur Inline-Überwachung breiter, schnell durchlaufender Papier- und Kunststoffbahnen.

Die Verwendung der Erfindung ist jedoch nicht auf ASOPS-THz-Spektrometer beschränkt. Sie ist auch in ähnlichen Anordnungen, z. B. ECOPS-Spektrometern, verwendbar.

Die in der Beschreibung und im Ausführungsbeispiel dargestellten Lösungen können in vielfacher Weise durch den Fachmann abgewandelt werden. Z. B. ist es nicht zwingend erforderlich, mit zeitlich äquidistanten Pulsabständen zu arbeiten. Der Fachmann kann auf der Basis der oben bereitgestellten Informationen ohne Weiteres die Pulsabstände so variieren, dass sie seiner konkreten Anwendung entsprechen. Auch diese und alle weiteren für den Fachmann naheliegenden Varianten der Erfindung, z. B. die Verwendung von Pulsen mit unterschiedlichen Intensitäten, fallen unter den Schutzumfang dieser Anmeldung.

**Abbildungslegenden**

Fig. 1 (Stand der Technik)

Fig. 1a: Fast leerer Zeitbereich eines herkömmlichen ASOPS-THz-Spektrometers, Fig. 1b: Unvollständige Füllung des Zeitbereichs eines ASOPS-THz-Spektrometers gemäß EP14156712.3.

Fig. 2a: Unvollständige Füllung des Zeitbereichs eines ASOPS-THz-Spektrometers nach Grobzerlegung eines fs-IR-Laserpulses.

Fig. 2b: Vollständige Füllung des Zeitbereichs eines ASOPS-THz-Spektrometers nach Grobzerlegung eines fs-IR-Laserpulses und Feinzerlegung der generierten THz-Pulse.

Fig. 3a: Neuartige Optik mit HDPE-Blöcken zur Feinzerlegung eines THz-Pulses in THz-Subpulse.

Fig. 3b: Experimentelle Ergebnisse zur Linearisierung der zeitlichen Abstände der THz-Subpulse.

Fig. 4: Schematische Darstellung eines erfindungsgemäßen ASOPS-THz-Spektrometers.

**Patentansprüche**

1. Verfahren zur Füllung eines Zeitbereichs Z eines THz-Zeitbereichsspektrometers, insbesondere eines ASOPS-THz-Spektrometers oder eines ECOPS-THz-Spektrometers, mit Signalen in Form von THz-Subpulsen, **dadurch gekennzeichnet, dass** im optischen Pfad des Zeitbereichsspektrometers eine Grobzerlegung von IR-Anregungspulsen in M zeitversetzte IR-Subpulse erfolgt, welche zu M THz-Senderantennen ($S_1,...,S_M$) geführt werden und diese zur Emission von M THz-Pulsen anregen, wobei jeder THz-Puls einer Feinzerlegung in N zeitversetzte THz-Subpulse unterzogen wird, sodass aus jedem IR-Anregungspuls MxN THz-Subpulse erzeugt werden, die das THz-Spektrometer auf räumlich separierten Strahlwegen durchlaufen und dann als möglichst lückenlose Folge von sich nicht überlagernden THz-Subpulsen auf eine THz-Empfängerantenne E geführt werden.

2. Verfahren nach Anspruch 1, **dadurch gekennzeichnet, dass** die Grobzerlegung der IR-Anregungspulse in M zeitversetzte IR-Subpulse realisiert wird, indem die IR-Subpulse fasergekoppelt auf Strahlwegen mit definierten Längendifferenzen $\Delta L$ zu den M THz-Senderantennen geführt werden.

3. Verfahren nach Anspruch 1 oder 2, **dadurch gekennzeichnet, dass** die Feinzerlegung jedes THz-Pulses in N zeitversetzte THz-Subpulse realisiert wird, indem jeder THz-Puls in Wechselwirkung mit einer senderseitigen THz-Optik ($K_1, K_2,...; B_1, B_2,...$) tritt, wobei unterschiedlich lange Strahlwege innerhalb der THz-Optik mit einem Brechungsindex n, der größer ist als der Brechungsindex der Umgebung nu, und/oder Reflexionen an inneren und äußeren Oberflächen der THz-Optik ausgenutzt werden.

4. Verfahren nach einem der Ansprüche 1 bis 3, **dadurch gekennzeichnet, dass** zur Feinanpassung der zeitlichen Abstände in der Folge von THz-Subpulsen eine Längenanpassung der fasergekoppelten Strahlwege und/oder eine örtliche Versetzung der Positionen der M THz-Senderantennen ($S_1,...,S_M$) und/oder eine Anpassung der empfängerseitigen Optik (O) erfolgt.

5. Verfahren nach Anspruch 4, **dadurch gekennzeichnet, dass** für die M THz-Senderantennen ($S_1,...,S_M$) ein umso kürzerer fasergekoppelter Strahlweg und damit eine umso geringere Zeitverzögerung gewählt werden, je größer die Entfernung der jeweiligen THz-Senderantenne ($S_1,...,S_M$) von der THz-Empfängerantenne (E) ist.

6. Verfahren nach einem der Ansprüche 1 bis 5, **dadurch gekennzeichnet, dass** bei der Grobzerlegung eine geeignete Anzahl M $\geq$ 1 von IR-Subpulsen und/oder bei der Feinzerlegung eine geeignete Anzahl N $\geq$ 1 von THz-Subpulsen gewählt werden.

7. Verfahren nach einem der Ansprüche 1 bis 6, **dadurch gekennzeichnet, dass** durch die Kombination von Grobzerlegung und Feinzerlegung ein sehr großer Zeitbereich, bevorzugt 4 ns, besonders bevorzugt 10 ns, gleichmäßig mit THz-Subpulsen gefüllt wird.

8. Vorrichtung zur Realisierung eines THz-Zeitbereichsspektrometers, geeignet zur Durchführung eines Verfahrens nach einem der Ansprüche 1 bis 7, aufweisend einen senderseitigen Laser (Ls) und einen empfängerseitigen Laser ($L_E$), deren Repetitionsraten gegeneinander verstimmt sind, eine THz-Empfängerantenne (E), eine empfängerseitige Optik (O) sowie eine senderseitige Anordnung, die zwischen dem senderseitigen Laser (Ls) und einer Ebene, in der eine nicht als Teil des Spektrometers zu betrachtende Probe (Pr) anordenbar ist, positioniert ist, **dadurch gekennzeichnet, dass** die senderseitige Anordnung umfasst: M THz-Senderantennen ($S_1,...,S_M$), die über M Fasern ($Z_1,...,Z_M$) mit dem senderseitigen Laser (Ls) verbunden sind, sowie M THz-Optiken.

9. Vorrichtung nach Anspruch 8, **dadurch gekennzeichnet, dass** das THz-Zeitbereichsspektrometer als ASOPS-THz-Spektrometer oder als ECOPS-THz-Spektrometer ausgeführt ist.

10. Vorrichtung nach Anspruch 8 oder 9, **dadurch gekennzeichnet, dass** eine THz-Optik ausgeführt ist als Stufenkeil ($K_1,...,K_M$), aufweisend N linear angeordnete Stufen oder N zweidimensional auf einer Fläche angeordnete Stufen, mit vorgeschalteter THz-Linse ($L_1,...,L_M$), mit der ein von einer THz-Sen-

derantenne ($S_1$,...,$S_M$) ausgehendes divergentes THz-Signal auf die Abmessungen eines Stufenkeils ($K_1$,...,$K_M$) kollimiert wird.

11. Vorrichtung nach Anspruch 8 oder 9, **dadurch gekennzeichnet, dass** eine THz-Optik ausgeführt ist als Blockanordnung ($B_1$, $B_2$,...), mit der ein THz-Strahl in räumlich getrennte THz-Subpulse ($P_{1,1}$, $P_{1,2}$,...) separiert wird.

12. Vorrichtung nach Anspruch 11, **dadurch gekennzeichnet, dass** die als Blockanordnung ($B_1$, $B_2$,...) ausgeführte THz-Optik mit einem Spiegelsystem zur Linearisierung der zeitlichen Abstände von THz-Subpulsen ausgestattet ist.

13. Vorrichtung nach einem der Ansprüche 8 bis 12, **dadurch gekennzeichnet, dass** die M Fasern ($Z_1$,..., $Z_M$) als Zero Dispersion Fibers ausgeführt sind.

14. Vorrichtung nach Anspruch 13, **dadurch gekennzeichnet, dass** gleichlange Abschnitte (Lo) der M Zero Dispersion Fibers durch herkömmliche optische Fasern ersetzt sind.

15. Verwendung eines Verfahrens gemäß einem der Ansprüche 1 bis 7 sowie einer Vorrichtung gemäß einem der Ansprüche 8 bis 14 zur Kontrolle industrieller Fertigungsprozesse oder zur Inline-Überwachung breiter, schnell durchlaufender Papier- und Kunststoffbahnen.

Fig. 1a

Fig. 1b

Fig. 2a

Fig. 2b

Fig. 3a

Fig. 3b

t/ns

Index j

Fig. 4

**Europäisches Patentamt**
**European Patent Office**
**Office européen des brevets**

# EUROPÄISCHER RECHERCHENBERICHT

**Nummer der Anmeldung**

EP 15 00 2578

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2007/158571 A1 (COLE BRYAN E [GB] ET AL) 12. Juli 2007 (2007-07-12) | 8,9,12, 15 | INV. G01N21/3586 |
| Y | * Absatz [0001] - Absatz [0002]; Abbildungen 1,11,12 * <br> * Absatz [0010] * <br> * Absatz [0050] * <br> * Absatz [0076] - Absatz [0080] * <br> ----- | 1-7,10, 11,13-15 | G01N33/34 G01N21/86 G01N21/89 |
| Y | WO 2004/072593 A2 (PHILIP MORRIS PROD [CH]; TRAN PHUC G [US]; SHAFER KENNETH H [US]) 26. August 2004 (2004-08-26) <br> * Seite 2, Zeile 13 - Zeile 22; Abbildung 1 * <br> * Seite 3, Zeile 17 - Seite 4, Zeile 1 * <br> * Seite 5, Zeile 1 - Zeile 4 * <br> * Seite 7, Zeile 3 - Zeile 5 * <br> * Seite 8, Zeile 3 - Zeile 23 * <br> * Seite 9, Zeile 4 - Zeile 13 * <br> ----- | 1-7,10, 15 | |
| Y | MAN ZHANG ET AL: "A Compressed Terahertz Imaging Method", CHINESE PHYSICS LETTERS, Bd. 29, Nr. 10, Oktober 2012 (2012-10), Seiten 104208-1, XP055127644, GB ISSN: 0256-307X, DOI: 10.1088/0256-307X/29/10/104208 <br> * Seite 1, rechte Spalte, Zeile 5 - Zeile 14; Abbildung 1 * <br> ----- | 3,10 | **RECHERCHIERTE SACHGEBIETE (IPC)** <br><br> G01N <br> H01S <br> G01J |
| Y | EP 2 538 186 A1 (UNIV MARBURG PHILIPPS [DE]) 26. Dezember 2012 (2012-12-26) <br> * Absatz [0010] * <br> * Absatz [0026] * <br> ----- | 3 | |
| Y | EP 2 031 375 A2 (CANON KK [JP]) 4. März 2009 (2009-03-04) <br> * Absatz [0028]; Abbildung 1 * <br> ----- | 3,11 | |

-/--

Der vorliegende Recherchenbericht wurde für alle Patentansprüche erstellt

| Recherchenort | Abschlußdatum der Recherche | Prüfer |
|---|---|---|
| München | 29. Februar 2016 | Witte, Thomas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

European Patent Office

Office européen des brevets

**EUROPÄISCHER RECHERCHENBERICHT**

Nummer der Anmeldung

EP 15 00 2578

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | EP 2 621 031 A2 (MENLO SYSTEMS GMBH [DE]) 31. Juli 2013 (2013-07-31) <br> * Absatz [0004]; Abbildung 1 * <br> * Absatz [0037] * <br> * Absatz [0065] - Absatz [0067] * <br> ----- | 13,14 | |
| A | BARTELS A ET AL: "Femtosecond time-resolved optical pump-probe spectroscopy at kilohertz-scan-rates over nanosecond-time-delays without mechanical delay line", <br> APPLIED PHYSICS LETTERS, AMERICAN INSTITUTE OF PHYSICS, 2 HUNTINGTON QUADRANGLE, MELVILLE, NY 11747, <br> Bd. 88, Nr. 4, <br> 25. Januar 2006 (2006-01-25), Seiten 41117-041117, XP012082496, <br> ISSN: 0003-6951, DOI: 10.1063/1.2167812 <br> * Seiten 041117-1 * <br> ----- | 1,8 | |
| A | YOUNGCHAN KIM AND DAE-SU YEE: "High-speed terahertz time-domain spectroscopy based on electronically controlled optical sampling", <br> OPTICS LETTERS, OPTICAL SOCIETY OF AMERICA, US, <br> Bd. 35, Nr. 22, <br> 15. November 2010 (2010-11-15), Seiten 3715-3717, XP001559083, <br> ISSN: 0146-9592, DOI: 10.1364/OL.35.003715 <br> * Seite 3715 * <br> ----- <br> -/-- | 1,8 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 29. Februar 2016 | Witte, Thomas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument

EPO FORM 1503 03.82 (P04C03)

Europäisches Patentamt

European Patent Office

Office européen des brevets

Nummer der Anmeldung

EP 15 00 2578

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | THOMAS HOCHREIN ET AL: "Optical sampling by laser cavity tuning", OPTICS EXPRESS, Bd. 18, Nr. 2, 18. Januar 2010 (2010-01-18), Seite 1613, XP055250842, ISSN: 2161-2072, DOI: 10.1364/OE.18.001613 * Seite 1613 * | 1,8 | |
| A | DE 10 2006 012715 A1 (UNIV BRAUNSCHWEIG TECH [DE]; JENA OPTRONIK GMBH [DE]) 20. September 2007 (2007-09-20) * das ganze Dokument * | 1 | |
| A | P.U. JEPSEN ET AL: "Terahertz spectroscopy and imaging - Modern techniques and applications", LASER & PHOTONICS REVIEWS, Bd. 5, Nr. 1, 4. Oktober 2010 (2010-10-04), Seiten 124-166, XP055127649, ISSN: 1863-8880, DOI: 10.1002/lpor.201000011 * Seite 153 - Seite 158 * | 15 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 29. Februar 2016 | Witte, Thomas |

**ANHANG ZUM EUROPÄISCHEN RECHERCHENBERICHT**
**ÜBER DIE EUROPÄISCHE PATENTANMELDUNG NR.**

EP 15 00 2578

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-02-2016

| Im Recherchenbericht angeführtes Patentdokument | Datum der Veröffentlichung | Mitglied(er) der Patentfamilie | Datum der Veröffentlichung |
|---|---|---|---|
| US 2007158571 A1 | 12-07-2007 | GB 2411093 A<br>US 2007158571 A1<br>WO 2005080947 A1 | 17-08-2005<br>12-07-2007<br>01-09-2005 |
| WO 2004072593 A2 | 26-08-2004 | US 2004155192 A1<br>US 2004155193 A1<br>WO 2004072593 A2 | 12-08-2004<br>12-08-2004<br>26-08-2004 |
| EP 2538186 A1 | 26-12-2012 | EP 2538186 A1<br>WO 2012175325 A1 | 26-12-2012<br>27-12-2012 |
| EP 2031375 A2 | 04-03-2009 | CN 101377465 A<br>EP 2031375 A2<br>JP 5144175 B2<br>JP 2009058310 A<br>US 2009056455 A1 | 04-03-2009<br>04-03-2009<br>13-02-2013<br>19-03-2009<br>05-03-2009 |
| EP 2621031 A2 | 31-07-2013 | DE 102012001357 A1<br>EP 2621031 A2<br>US 2013188661 A1 | 25-07-2013<br>31-07-2013<br>25-07-2013 |
| DE 102006012715 A1 | 20-09-2007 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 14156712 A

**In der Beschreibung aufgeführte Nicht-Patentliteratur**

- **A. BARTELS ; T. DEKORSY.** *Techn. Messen,* 2008, vol. 75, 1-8